(19) Europäisches Patentamt / European Patent Office / Office européen des brevets

(11) EP 3 201 184 B1

(12) **EUROPÄISCHE PATENTSCHRIFT**

(45) Veröffentlichungstag und Bekanntmachung des Hinweises auf die Patenterteilung:
**02.01.2019 Patentblatt 2019/01**

(21) Anmeldenummer: **15770541.9**

(22) Anmeldetag: **28.09.2015**

(51) Int Cl.:
***C07D 311/92*** *(2006.01)* ***C12P 17/06*** *(2006.01)*

(86) Internationale Anmeldenummer:
**PCT/EP2015/072262**

(87) Internationale Veröffentlichungsnummer:
**WO 2016/050690 (07.04.2016 Gazette 2016/14)**

(54) **VERFAHREN ZUR BIOKATALYTISCHEN CYCLISIERUNG VON GERANYLLINALOOL UND DABEI ERHALTENE CYCLISIERUNGSPRODUKTE**

METHOD FOR THE BIOCATALYTIC CYCLISATION OF GERANYLLINALOOL AND RESULTING CYCLISATION PRODUCTS

PROCÉDÉ DE CYCLISATION BIO-CATALYTIQUE DE GÉRANYL-LINALOOL ET PRODUITS CYCLISÉS AINSI OBTENUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.09.2014 EP 14186830**

(43) Veröffentlichungstag der Anmeldung:
**09.08.2017 Patentblatt 2017/32**

(73) Patentinhaber: **BASF SE**
**67056 Ludwigshafen (DE)**

(72) Erfinder:
- **BREUER, Michael**
  **64285 Darmstadt (DE)**
- **PELZER, Ralf**
  **37699 Fürstenberg (DE)**

(74) Vertreter: **BASF IP Association**
**BASF SE**
**ZRX-C6**
**67056 Ludwigshafen (DE)**

(56) Entgegenhaltungen:
**WO-A2-2012/066059**

- **GABRIELE SIEDENBURG ET AL: "Squalene-Hopene Cyclases", APPLIED AND ENVIRONMENTAL MICROBIOLOGY, Bd. 77, Nr. 12, 2011, Seiten 3905-3915, XP055156447, ISSN: 0099-2240, DOI: 10.1128/AEM.00300-11 in der Anmeldung erwähnt**
- **TADAHIRO KATO ET AL: "Brominative cyclisation of nerolidol and geranyl-linalool", JOURNAL OF THE CHEMICAL SOCIETY, CHEMICAL COMMUNICATIONS, Nr. 23, 1980, Seiten 1106-1108, XP055156268, ISSN: 0022-4936, DOI: 10.1039/c39800001106**
- **NILS GÜNNEWICH ET AL: "A diterpene synthase from the clary sage Salvia sclarea catalyzes the cyclization of geranylgeranyl diphosphate to (8R)-hydroxy-copalyl diphosphate", PHYTOCHEMISTRY, Bd. 91, 2013, Seiten 93-99, XP002724040, ISSN: 0031-9422, DOI: 10.1016/J.PHYTOCHEM.2012.07.019**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neuartiges Verfahren zur Cyclisierung von Geranyllinalool unter Verwendung der aus *Zymomonas mobilis* stammenden Squalen-Hopen-Cyclase (Zm-SHC) oder einer Cyclase mit wenigstens 95 % Sequenzidentität zur Zm-SHC..

## Hintergrund der Erfindung

Die Biosynthese von zahlreichen Monoterpenen in den entsprechenden Produktionsorganismen wurde bereits aufgeklärt. Häufig werden dabei lineare Vorläufermoleküle durch hochspezifische Biokatalysatoren zyklisiert. Bei den Vorläufern handelt es sich in der Regel um Ester linearer Terpenalkohole und Diphosphorsäure. Ein typisches Beispiel für eine solche Vorstufe ist Geranylpyrophosphat. Die Pyrophosphatgruppe wird enzymatisch aus dem Molekül eliminiert und anschließend in zwei Phosphationen hydrolysiert. Auf der anderen Seite entsteht dabei ein Carbokation, das nun intramolekular weiterreagieren kann und, z.B. unter Abspaltung eines Protons, zu einem zyklischen Monoterpen rekombiniert (Curr. Opin. Chem. Biol. 2009, 13:180-188).

Bekanntlich werden *in vivo* nicht-aktivierte Triterpene wie Squalen oder Oxidosqualen durch Squalen-Hopen-Cyclasen (SHC) zu den entsprechenden zyklischen Verbindungen umgesetzt (Siedenburg, G. und Jendrossek, D., Applied and Environmental Microbiology, 2011, 77, (12), 3905).

Die Aktivität bestimmter Squalen-Hopen-Cyclasen (SHC) ist nicht auf Triterpene beschränkt. In der internationalen Anmeldung PCT/EP2011/070304 (WO 2012066059 A2), auf deren vollständige Offenbarung hierin ausdrücklich Bezug genommen wird, werden Squalen-Hopen Cyclase-Mutanten beschrieben, welche die Cyclisierung eines Citronellal-Isomers zu einem Isopulegol-Isomer katalysieren.

In der internationalen Anmeldung PCT/EP2010/057696 (WO2010139719 A2), auf deren vollständige Offenbarung hierin ausdrücklich Bezug genommen wird, werden Squalen-Hopen-Cyclasen als Biokatalysatoren für die Cyclisierung von Homofarnesol zu Ambroxan vorgeschlagen.

Die Gen- und Proteinsequenzen der aus dem Bakterium *Zymomonas mobilis* stammenden Squalen-Hopen-Cyclase (Zm-SHC) sind bekannt (Genpept Accession No AAV90172 2004 und Nat Biotechnol 2005, 23:63-68, vgl. SEQ ID NO:1 und 2).

Es war Aufgabe der vorliegenden Erfindung, ein Verfahren zur Cyclisierung von Geranyllinalool bereitzustellen.

## Kurzfassung der Erfindung

Obige Aufgabe wurde durch ein Verfahren zur biokatalytischen Cyclisierung einer Verbindung der Formel (I)

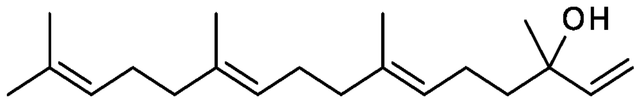

(I)

in Gegenwart einer Cyclase, die eine Aminosäuresequenz gemäß SEQ ID NO:2 oder mit wenigstens 95 % Sequenzidentität zu SEQ ID NO:2 aufweist, gelöst.

In dem erfindungsgemäßen Verfahren setzt man die Verbindung der Formel (I) zu wenigstens einer Verbindung der Formel (II)

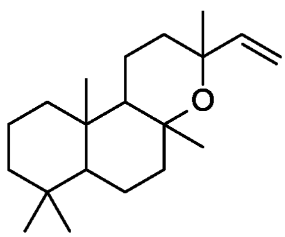

(II)

um.

## Detaillierte Beschreibung der Erfindung

### A. Allgemeine Definitionen

"Cyclasen" im Sinne der vorliegenden Erfindung sind allgemein Enzyme bzw. Enzymmutanten, welche insbesondere die Aktivität einer Geranyllinalool-Cyclase zeigen. Unter der Aktivität einer Geranyllinalool-Cyclase wir eine enzymatische Aktivität zur Isomerisierung von Geranyllinalool (I) unter Ausbildung mindestens eines 5- oder 6-gliedrigen Rings, insbesondere einer Chromen-artigen und vor allem einer Benzochromen-artigen cyclisierten Struktur verstanden. Als Enzyme mit der Aktivität einer Geranyllinalool-Cyclase sind intramolekulare Transferasen aus der Subklasse der Isomerasen; also Proteine mit der EC-Nummer EC 5.4 geeignet. (Enzymcode gemäß Eur. J. Biochem. 1999, 264, 610-650). Insbesondere handelt es sich um Vertreter von EC 5.4.99.17. Als Enzyme mit der Aktivität einer Geranyllinalool-Cyclase sind insbesondere solche Cyclasen geeignet, die auch die Cyclisierung von Homofarnesol zu Ambroxan, von einem Citronellal- zu einem Isopulegol-Isomer oder von Squalen zu Hopen (daher auch zuweilen die Bezeichnung "SHC" Squalen Hopen Cyclase) bewirken und die ausführlich in den internationalen Anmeldungen PCT/EP2010/057696 und PCT/EP2011/070304 beschrieben werden, auf die hier ausdrücklich Bezug genommen wird.

Aufgrund der Reversibilität enzymatischer Reaktionen betrifft die vorliegende Erfindung die hierin beschriebenen enzymatischen Umsetzungen in beiden Umsetzungsrichtungen.

"Funktionale Mutanten" einer "Cyclase" umfassen die unten definierten "funktionalen Äquivalente" solcher Enzyme.

Der Begriff "biokatalytisches Verfahren" betrifft jegliches "Verfahren zur biokatalytischen Cyclisierung einer Verbindung der Formel (I)", d.h. Verfahren in Gegenwart von rohem, gereinigtem, gelöstem, dispergiertem oder immobilisiertem Enzym, oder in Gegenwart von Cyclase aufweisenden Zellen eines Mikroorganismus, welche die Aktivität einer Geranyllinalool-Cyclase aufweisen oder exprimieren. Biokatalytische Verfahren umfassen somit enzymatische als auch mikrobielle Verfahren sowie fermentative Verfahren.

Der Begriff "stereospezifisch" bedeutet, dass eines von mehreren möglichen Stereoisomeren einer erfindungsgemäß hergestellten Verbindung mit wenigstens einem Asymmetriezentrum durch die Wirkung eines erfindungsgemäß eingesetzten Enzyms in hohem "Enatiomerenüberschuß" oder hoher "Enantiomerenreinheit", wie z.B. wenigstens 90 %ee, insbesondere wenigstens 95 %ee, oder wenigstens 98 %ee, oder wenigstens 99 %ee produziert wird. Der ee% Wert wird nach folgender Formel berechnet:

$$ee\% = [X_A - X_B]/[X_A + X_B]*100,$$

worin $X_A$ und $X_B$ für den Molenbruch der Enantiomere A bzw B stehen.

"Erste-Sphäre-Reste" und "Zweite-Sphäre-Reste" sind Aminosäurereste, denen, basierend auf Strukturanalysen des Proteins, eine besondere Nähe zum reaktiven Zentrum der Cyclase zugeordnet wird. Das Kriterium für die erste Sphäre ist die Distanz zum Liganden 2-Azasqualen, der in einer publizierten Röntgenstruktur angegeben ist (pdb: 1ump). Diese Reste wurden automatisch mit einem Computerprogramm ermittelt (http://ligin.weizmann.ac.il/cgibin/lpccsu/LpcCsu.cgi; Sobolev V, Sorokine A, Prilusky J, Abola EE, Edelman M. Automated analysis of interatomic contacts in proteins. Bioinformatics 1999;15(4):327-332.). Dieses Programm geht davon aus, dass zwei Moleküle in Kontakt miteinander stehen wenn die Entfernung ihrer Atome der Summe ihrer van der Waals Radien ± 1 Å entspricht. Zur zweiten Sphäre werden alle Aminosäuren gezählt, die in einem Radius von 5 Å zu jedem Rest der ersten Sphäre liegen. Derartige Reste sind daher für die Vornahme von gezielten Mutationen besonders geeignet, um die Enzymaktivität weiter gezielt zu modifizieren.

Eine "Cyclase-Aktivität", die mit Geranyllinalool der Formel (I), insbesondere E,E Geranyllinalool, als Referenzsubstrat unter Standardbedingungen bestimmt wurde, ist eine Enzymaktivität, welche die Bildung eines cyclischen Reaktionsprodukts beschreibt.

"Standardbedingungen" sind z.B. Substratkonzentrationen von 10 mM bis 0,2 M, insbesondere 15 bis 100 mM, wie z.B. etwa 20 bis 25 mM; bei pH 4 bis 8, und bei Temperaturen von z.B. 15 bis 45 oder 20 bis 25 °C. Die Bestimmung kann dabei mit rekombinanten Cyclase-exprimierenden Zellen, aufgeschlossenen Cyclase-exprimierenden Zellen, Fraktionen davon oder angereichertem oder gereinigtem Cyclase-Enzym erfolgen. Insbesondere ist Referenzsubstrat ein Geranyllinalool der Formel (I); insbesondere E,E-Geranyllinalool in einer Konzentration von 15 bis 100 mM oder etwa 20 bis 25 mM, bei 20 bis 25 °C und pH 6-7, wie etwa 6,5; wie auch in den Bespielen näher beschrieben.

Generell mit umfasst sind erfindungsgemäß alle isomeren Formen der hierin beschriebenen Verbindungen, wie Konstitutionsisomere und insbesondere Stereoisomere und Gemische davon, wie z.B. optische Isomere oder geometrische Isomere, wie E- und Z-Isomere, sowie Kombinationen davon. Sind mehrere Asymmetriezentren in einem Molekül vorhanden, so umfasst die Erfindung sämtliche Kombinationen von unterschiedlichen Konformationen dieser Asymmetriezentren, wie z.B. Enantiomerenpaare oder jegliche Mischungen von stereoisomeren Formen.

**B. Spezielle Ausgestaltungen der Erfindung**

Die vorliegende Erfindung betrifft insbesondere folgende spezielle Ausführungsformen:

1. Verfahren zur biokatalytischen Cyclisierung einer Verbindung der Formel (I)

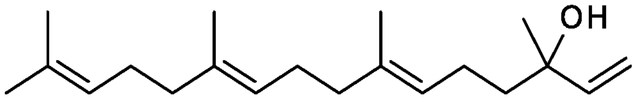

(I)

in Gegenwart einer Cyclase, die eine Aminosäuresequenz gemäß SEQ ID NO:2 oder mit wenigstens 95 %, wie z.B. 96 %, 97 %, 98 % oder 99 % Sequenzidentität zu SEQ ID NO:2 aufweist;
wobei man die Verbindung der Formel (I) zu der Verbindung der Formel (II)

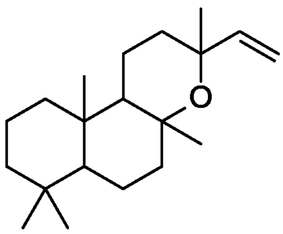

(II),

welche insbesondere in stereoisomerenreiner Form sowie als Gemisch umfassend wenigstens eines der möglichen Stereoisomere dieser Verbindung vorliegt, umsetzt.

2. Verfahren nach Ausführungsform 1, wobei die Verbindung der Formel (II) in Form eines Gemischs, das mehrere Stereoisomere umfasst, vorliegt.

3. Verfahren nach einer der vorhergehenden Ausführungsformen, wobei die Verbindung der Formel (II) in Form eines Stereoisomers vorliegt.

4. Verfahren nach einem der vorhergehenden Ausführungsformen, wobei man wenigstens eine Verbindung der Formel (I) in einem flüssigen, insbesondere in einem einphasig wässrigem oder in einem zweiphasig wässrig-organischem Reaktionsmedium mit der Cyclase in Kontakt bringt, insbesondere unter Bedingungen welche die gewünschte Reaktion nicht beeinträchtigen, und vor allem unter Bedingungen, welche die gewünschte Reaktion begünstigen. Geeignete Reaktionsbedingungen (wie z.B. optimale Konzentration an Substrat, Enzym, pH-Wert, Art des ggf. verwendeten Puffers, Reaktionstemperatur und -dauer, organisches Lösungsmittel) können vom Durchschnittsfachmann durch wenige Vorversuche ohne weiteres bestimmt werden.

5. Verfahren nach einer der vorhergehenden Ausführungsformen, wobei man die Verbindung der Formel (I) in Form eines Gemisches beider Enantiomere einsetzt.

6. Verfahren nach Ausführungsform 5, wobei das Gemisch ein racemisches Gemisch ist.

7. Verfahren nach Ausführungsform 5, wobei eines der beiden Enantiomere in dem Gemisch im Überschuss vorliegt.

8. Verfahren nach einer der vorhergehenden Ausführungsformen, wobei die Cyclase roh, gereinigt, gelöst, dispergiert oder immobilisiert vorliegt, oder in Gegenwart von Cyclase aufweisenden Zellen eines Mikroorganismus.

9. Verfahren nach einer der vorhergehenden Ausführungsformen, wobei die Cyclase in einer Form vorliegt, ausgewählt unter:

a) freier, gegebenenfalls gereinigter oder teilweise gereinigter Cyclase;
b) immobilisierter Cyclase;
c) aus Zellen isolierter Cyclase gemäß a) oder b);
d) ganzen gegebenenfalls rekombinanten, Zellen, gegebenenfalls ruhenden oder aufgeschlossenen Cyclase-enthaltenden Zellen;
e) Zelllysat oder Zellhomogenisat der Zellen beschrieben unter d).

10. Verfahren nach einer der vorhergehenden Ausführungsformen in Gegenwart eines rekombinanten Mikroorganismus, der eine für die Cyclase kodierende Nukleotidsequenz trägt.

11. Verfahren nach Ausführungsform 10, wobei die Nukleotidsequenz Teil einer Expressionskassette ist und darin unter der Kontrolle wenigstens einer regulativen Sequenz steht.

12. Verfahren nach Ausführungsform 11, wobei die Expressionskassette Teil eines Expressionsvektors ist.

13. Verfahren nach Ausführungsform 12, wobei der Expressionsvektor unter Plasmiden ausgewählt ist.

14. Verfahren nach einer der vorhergehenden Ausführungsformen, wobei der Mikroorganismus unter Bakterien, Pilzen und Hefen ausgewählt ist.

15. Verfahren nach einer der Ausführungsformen 4 bis 15, wobei der Mikroorganismus unter den *Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis* und *Zymomonas mobilis* ausgewählt ist.

16. Verfahren nach einer der vorhergehenden Ausführungsformen, wobei der Mikroorganismus *E. coli* ist.

17. Verfahren nach einer der vorhergehenden Ausführungsformen, wobei die biokatalytische Cyclisierung in einphasigen wässrigen Systemen oder in zweiphasigen Systemen erfolgt; oder in wasserfreien Systemen, wie in ionischen Flüssigkeiten oder sog. *deep eutectic solvents.*

18. Verfahren nach einer der vorhergehenden Ausführungsformen, wobei die biokatalytische Cyclisierung bei einer Temperatur im Bereich von 20 bis 45°C und/oder einem pH-Wert im Bereich von 4 bis 8 erfolgt.

### C. Weitere Ausgestaltungen der Erfindung

### 1. Im erfindungsgemäßen Verfahren eingesetzte Cyclasen

Die vorliegende Erfindung ist nicht auf Verfahren beschränkt, in denen die hierin konkret offenbarte Cyclase eingesetzt wird, sondern erstreckt sich vielmehr auch auf Verfahren, durchgeführt unter Verwendung funktionaler Äquivalente der konkret beschriebenen Cyclase.

"Funktionale Äquivalente" oder Analoga der konkret offenbarten Enzyme und Enzymmutanten (abgeleitet von SEQ ID NO:2) sind im Rahmen der vorliegenden Erfindung davon verschiedene Polypeptide, welche weiterhin die gewünschte Cyclase-Aktivität besitzen.

So versteht man beispielsweise unter "funktionalen Äquivalenten" Enzyme und Mutanten, die in einem verwendeten Test auf "Cyclase-Aktivität" im Sinne der Erfindung (d.h. mit einem Referenzsubstrat unter Standardbedingungen) eine um mindestens 1 %, insbesondere um mindestens etwa 5 bis 10 % wie z.B. mindestens 10 % oder mindestens 20 %, wie z.B. mindestens 50 % oder 75 % oder 90 % höhere oder niedrigere Aktivität eines Enzyms, umfassend eine hierin konkret definierte Aminosäuresequenz abgeleitet von SEQ ID NO:2 aufweisen.

Die Aktivitätsangaben für funktionale Äquivalente beziehen sich hierin, wenn nichts anderes angegeben ist, auf Aktivitätsbestimmungen, durchgeführt mittels eines Referenzsubstrates (unter "Standardbedingungen" wie hierin definiert).

Die "Cyclase-Aktivität" im Sinne der Erfindung kann mit Hilfe eines Test unter Verwendung des Referenzsubstrates, wie z. B. Geranyllinalool, unter Standardbedingungen, wie oben beschrieben und im experimentellen Teil erläutert, bestimmt werden.

Funktionale Äquivalente sind außerdem z. B. zwischen pH 4 bis 11 stabil und besitzen vorteilhaft ein pH-Optimum in einem Bereich von pH 5 bis 10, wie insbesondere 6,5 bis 9,5 oder 7 bis 8 oder etwa bei 7,5, sowie ein Temperaturoptimum im Bereich von 15 °C bis 80 °C oder 20 °C bis 70 °C, wie z.B. etwa 30 bis 60 °C oder etwa 35 bis 45 °C, wie etwa bei 40 °C.

"Funktionale Äquivalente" im obigen Sinne sind auch "Präkursoren" der beschriebenen Polypeptide sowie "funktionale Derivate" und "Salze" der Polypeptide.

"Funktionale Äquivalente" umfassen die durch eine oder mehrere, wie z. B. 1 bis 50, 2 bis 30, 2 bis 15, 4 bis 12 oder 5 bis 10 "Mutationen", wie Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d. h. beispielsweise gleiche Substrate mit unterschiedlicher Geschwindigkeit umgesetzt werden.

Nichtlimitierende Beispiele für geeignete Aminosäuresubstitutionen sind in folgender Tabelle zusammengefasst:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn; Gln |
| Ile | Leu; Val |
| Leu | Ile; Val |
| Lys | Arg; Gln; Glu |
| Met | Leu; Ile |
| Phe | Met; Leu; Tyr |
| Ser | Thr |
| Thr | Ser |
| Trp | Tyr |
| Tyr | Trp ; Phe |
| Val | Ile; Leu |

"Präkursoren" sind dabei natürliche oder synthetische Vorstufen der Polypeptide mit oder ohne der gewünschten biologischen Aktivität.

Unter dem Ausdruck "Salze" versteht man sowohl Salze von Carboxylgruppen als auch Säureadditionssalze von Aminogruppen der Cyclase. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure werden ebenfalls erfindungsgemäß verwendet.

"Funktionale Derivate" der Cyclase können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der Cyclase, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitige wesentliche funktionelle Beeinträchtigung der Fusionsproteinteile) aufweisen. Nichtlimitierende Beispiele für derartige heterologe Sequenzen sind z.B. Signalpeptide, Histidin-Anker oder Enzyme.

Im Falle einer möglichen Proteinglykosylierung umfassen die Cyclasen "funktionale Äquivalente" in deglykosylierter bzw. glykosylierter Form sowie durch Veränderung des Glykosylierungsmusters erhältliche abgewandelte Formen.

Homologe der Cylase können durch Mutagenese erzeugt werden, z.B. durch Punktmutation, Verlängerung oder Verkürzung des Proteins.

Homologe der erfindungsgemäß eingesetzten Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäß verwendeter Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Erfindungsgemäß mit eingesetzte "funktionale Äquivalente" sind Homologe zu den konkret offenbarten Proteinen. Diese besitzen wenigstens 60 %, vorzugsweise wenigstens 75 % ins besondere wenigsten 85 %, wie z.B. 90, 91, 92, 93, 94, 95, 96, 97,98 oder 99 %, Homologie (bzw. Identität) zu einer der konkret offenbarten Aminosäuresequenzen, berechnet nach dem Algorithmus von Pearson und Lipman, Proc. Natl. Acad, Sci. (USA) 85(8), 1988, 2444-2448. Eine prozentuale Homologie bzw. Identität eines erfindungsgemäß verwendeten homologen Polypeptids bedeutet insbesondere prozentuale Identität der Aminosäurereste bezogen auf die Gesamtlänge einer der hierin konkret beschriebenen Aminosäuresequenzen.

Die prozentualen Identitätswerte können auch anhand von BLAST Alignments, Algorithmus blastp (protein-protein BLAST), oder durch Anwendung der unten angegebenen Clustal Einstellungen ermittelt werden.

## 2. Nukleinsäuren und Konstrukte

### 2.1 Nukleinsäuren

Das erfindungsgemäße Verfahren kann in Gegenwart eines Mikroorganismus durchgeführt werden, der eine für die Cyclase kodierende Nukleotidsequenz, also ein Nukleinsäuremolekül, trägt.

Alle hierin erwähnten Nukleinsäuremoleküle (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Es wird zwischen isolierten Nukleinsäuremolekülen, welche für die Cyclase oder biologisch aktive Abschnitte davon kodieren, und Nukleotidfragmenten, die z.B. zur Verwendung als Hybridisierungssonden oder Primer zur Identifizierung oder Amplifizierung von cyclasekodierenden Nukleinsäuren verwendet werden können, unterschieden.

Die Nukleinsäuremoleküle können zudem untranslatierte Sequenzen vom 3'- und/oder 5'-Ende des kodierenden Genbereichs enthalten.

Zu den konkret beschriebenen Nukleotidsequenzen existieren komplementäre Nukleinsäuremoleküle oder Abschnitte davon.

Die Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinander folgende Nukleotide eines Sense-Stranges der Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im Wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Das Nukleinsäuremolekül kann mittels molekularbiologischer Standard-Techniken und der bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die Oligonukleotide können ferner durch Standard-Syntheseverfahren, z.B. mit einem automatischen DNA-Synthesegerät, hergestellt werden.

Nukleinsäuresequenzen oder Derivate davon, Homologe oder Teile dieser Sequenzen, lassen sich beispielsweise mit üblichen Hybridisierungsverfahren oder der PCR-Technik aus anderen Bakterien, z.B. über genomische oder cDNA-Banken, isolieren. Diese DNA-Sequenzen hybridisieren unter Standardbedingungen mit den hierin beschriebenen Sequenzen.

Unter "hybridisieren" versteht man die Fähigkeit eines Poly- oder Oligonukleotids an eine nahezu komplementäre Sequenz unter Standardbedingungen zu binden, während unter diesen Bedingungen unspezifische Bindungen zwischen nicht-komplementären Partnern unterbleiben. Dazu können die Sequenzen zu 90-100 % komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze.

Zur Hybridisierung werden vorteilhaft kurze Oligonukleotide der konservierten Bereiche verwendet. Es können aber auch längere Fragmente der erfindungsgemäß eingesetzten Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wässrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42 °C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20 °C bis 45 °C, bevorzugt zwischen etwa 30 °C bis 45 °C. Für DNA:RNA-Hybride liegen die Hybridisierungsbedingungen vorteilhaft bei 0,1 x SSC und Temperaturen zwischen etwa 30 °C bis 55 °C, bevorzugt zwischen etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Die "Hybridisierung" kann insbesondere unter stringenten Bedingungen erfolgen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben.

Unter "stringenten" Hybridisierungs-Bedingungen werden insbesondere verstanden: Die Inkubation bei 42 °C über Nacht in einer Lösung bestehend aus 50 % Formamid, 5 x SSC (750 mM NaCl, 75 mM Tri-Natrium-citrat), 50 mM Natrium Phosphat (pH7,6), 5x Denhardt Lösung, 10 % Dextransulfat und 20 g/ml denaturierte, gescheerte Lachsspermien-DNA, gefolgt von einem Waschschritt der Filter mit 0,1x SSC bei 65 °C.

Die Nukleotidsequenzen können mit Promotoren fusioniert sein. Die Promotoren, die den angegebenen Nukleotidsequenzen vorgeschaltet sind, können durch wenigstens einen Nukleotidaustausch, wenigstens eine Insertion, Inversion und/oder Deletion verändert sein, ohne dass aber die Funktionalität bzw. Wirksamkeit der Promotoren beeinträchtigt sind. Des Weiteren können die Promotoren durch Veränderung ihrer Sequenz in ihrer Wirksamkeit erhöht oder komplett durch wirksamere Promotoren auch artfremder Organismen ausgetauscht werden.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

| Multiple alignment parameters: | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighting | 0 |

| Pairwise alignment parameter: | |
|---|---|
| FAST algorithm | on |
| K-tuplesize | 1 |
| Gap penalty | 3 |
| Window size | 5 |
| Number of best diagonals | 5 |

Alternativ dazu kann die Identität auch nach Chenna, Ramu, Sugawara, Hideaki, Koike,Tadashi, Lopez, Rodrigo, Gibson, Toby J, Higgins, Desmond G, Thompson, Julie D. Multiple sequence alignment with the Clustal series of programs. (2003) Nucleic Acids Res 31 (13):3497-500, gemäß Internetadresse: http://www.ebi.ac.uk/Tools/clustalw/index.html# und mit den folgenden Parametern bestimmt werden:

| | |
|---|---|
| DNA Gap Open Penalty | 15.0 |
| DNA Gap Extension Penalty | 6.66 |
| DNA Matrix | Identity |
| Protein Gap Open Penalty | 10.0 |
| Protein Gap Extension Penalty | 0.2 |
| Protein matrix | Gonnet |
| Protein/DNA ENDGAP | -1 |
| Protein/DNA GAPDIST | 4 |

### 2.2 Generierung funktionaler Mutanten

Dem Fachmann sind darüber hinaus Verfahren zur Erzeugung funktionaler Mutanten, also von Nukleotidsequenzen, die für eine Cyclase mit wenigstens 95 %, 96 %, 97 %, 98 % oder 99 % Sequenzidentität zu SEQ ID NO:2 kodieren, bekannt.

Je nach verwendeter Technik kann der Fachmann völlig zufällige oder auch gezieltere Mutationen in Gene oder auch nicht codierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Methoden zur Veränderung von Genen und somit zur Veränderung der von diesen codierten Protein sind dem

Fachmann seit langem geläufig, wie beispielsweise

- die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey),
- die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcárel R, Stunnenberg HG (1994) Nucleic Acids Res 22:541; Barik S (1995) Mol Biotechnol 3:1),
- die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739);
- die SeSaM-Methode (Sequence Saturation Method), bei der bevorzugte Austausche durch die Polymerase verhindert werden. Schenk et al., Biospektrum, Vol. 3, 2006, 277-279
- das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder
- das DNA-Shuffling, bei dem ein Pool aus nahe verwandten Genen gebildet und verdaut wird und die Bruchstücke als Templates für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747).

Unter Anwendung der so genannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme.

Die betreffenden Gene von Wirtsorganismen, die funktionale Mutanten mit Eigenschaften exprimieren, welche den gewünschten Eigenschaften weitgehend entsprechen, können einer weiteren Mutationsrunde unterworfen werden. Die Schritte der Mutation und der Selektion oder des Screening können iterativ solange wiederholt werden, bis die vorliegenden funktionalen Mutanten die gewünschten Eigenschaften in ausreichendem Maße aufweisen. Durch diese iterative Arbeitsweise können stufenweise eine begrenzte Anzahl von Mutationen, wie z.B. 1, 2, 3, 4 oder 5 Mutationen, vorgenommen und auf deren Einfluss auf die betreffende Enzymeigenschaft bewertet und selektiert werden. Die selektierte Mutante kann dann in gleicher Weise einem weiteren Mutationsschritt unterworfen werden. Dadurch lässt sich die Anzahl der zu untersuchenden Einzelmutanten signifikant verringern.

Die erfindungsgemäßen Ergebnisse liefern auch wichtige Informationen in Bezug auf Struktur und Sequenz der betreffenden Enzyme, die erforderlich sind, um gezielt weitere Enzyme mit gewünschten modifizierten Eigenschaften zu generieren. Insbesondere können sogenannte "hot spots" definiert werden, d.h. Sequenzabschnitte, die sich potentiell eignen, um über die Einführung gezielter Mutationen eine Enzymeigenschaft zu modifizieren.

Ebenfalls sind Informationen ableitbar bezüglich Aminosäure-Sequenzpositionen, in deren Bereich Mutationen durchgeführt werden können, die voraussichtlich wenig Einfluss auf die Enzymaktivität haben sollten, und als potentielle "silent mutations" bezeichnet werden können.

### 2.3 Konstrukte

In dem erfindungsgemäßen Verfahren kann die Nukleotidsequenz Teil einer Expressionskassette sein. Die Begriffe Expressionskassette und Expressionskonstrukt werden synonym verwendet. Der vorzugsweise rekombinante Expressionskonstrukt enthält unter der genetischen Kontrolle regulativer Nukleinsäuresequenzen eine für ein erfindungsgemäß eingesetztes Polypeptid kodierende Nukleinsäuresequenz.

In dem erfindungsgemäßen Verfahren kann die Expressionskassette Teil eines Expressionsvektors, insbesondere eines rekombinanten Expressionsvektors, sein.

Unter einer "Expressionseinheit" wird hierin eine Nukleinsäure mit Expressionsaktivität verstanden, die einen Promotor, wie hierein definiert umfasst, und nach funktioneller Verknüpfung mit einer zu exprimierenden Nukleinsäure oder einem Gen, die Expression, also die Transkription und die Translation dieser Nukleinsäure oder dieses Gens reguliert. Man spricht deshalb auch in diesem Zusammenhang von einer "regulativen Nukleinsäuresequenz". Zusätzlich zum Promotor können weitere, regulative Elemente, wie z.B. Enhancer, enthalten sein.

Unter einer "Expressionskassette" oder "Expressionskonstrukt" wird hierin eine Expressionseinheit verstanden, die mit der zu exprimierenden Nukleinsäure oder dem zu exprimierenden Gen funktionell verknüpft ist. Im Gegensatz zu einer Expressionseinheit umfasst eine Expressionskassette somit nicht nur Nukleinsäuresequenzen, welche Transkription und Translation regulieren, sondern auch die Nukleinsäuresequenzen, welche als Folge der Transkription und Translation als Protein exprimiert werden sollen.

Die Begriffe "Expression" oder "Überexpression" beschreiben im vorliegenden Kontext die Produktion bzw. Erhöhung der intrazellulären Aktivität eines oder mehrerer Enzyme in einem Mikroorganismus, die durch die entsprechende DNA kodiert werden. Dazu kann man beispielsweise ein Gen in einen Organismus einbringen, ein vorhandenes Gen durch ein anderes Gen ersetzen, die Kopienzahl des Gens bzw. der Gene erhöhen, einen starken Promotor verwenden oder ein Gen verwenden, das für ein entsprechendes Enzym mit einer hohen Aktivität kodiert und man kann gegebenenfalls diese Maßnahmen kombinieren.

Vorzugsweise umfassen solche hierin beschriebenen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einem "Promotor", einer "Nukleinsäure mit Promotoraktivität" oder einer "Promotorsequenz" wird eine Nukleinsäure verstanden, die in funktioneller Verknüpfung mit einer zu transkribierenden Nukleinsäure die Transkription dieser Nukleinsäure reguliert.

Unter einer "funktionellen" oder "operativen" Verknüpfung versteht man in diesem Zusammenhang beispielsweise die sequentielle Anordnung einer der Nukleinsäuren mit Promotoraktivität und einer zu transkribierenden Nukleinsäuresequenz und gegebenenfalls weiterer regulativer Elemente, wie zum Beispiel Nukleinsäuresequenzen, die die Transkription von Nukleinsäuren gewährleisten, sowie zum Beispiel einen Terminator, derart, dass jedes der regulativen Elemente seine Funktion bei der Transkription der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu transkribierende Nukleinsäuresequenz hinter (d.h. am 3'-Ende) der Promotorsequenz positioniert wird, so dass beide Sequenzen kovalent miteinander verbunden sind. Dabei kann der Abstand zwischen der Promotorsequenz und der transgen zu exprimierende Nukleinsäuresequenz geringer als 200 Basenpaare, oder kleiner als 100 Basenpaare oder kleiner als 50 Basenpaare sein.

Neben Promotoren und Terminator sind als Beispiele weiterer regulativer Elemente zu nennen Targeting-Sequenzen, Enhancer, Polyadenylierungssignale, selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Erfindungsgemäß verwendete Nukleinsäurekonstrukte umfassen insbesondere eine für eine Cyclase kodierende Sequenz, z.B. abgeleitet von SEQ ID NO: 1 oder kodierend für eine Cyclase nach SEQ ID NO: 2 oder Derivate und Homologe davon, sowie die davon ableitbaren Nukleinsäuresequenzen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäß verwendeten Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Beispiele geeigneter Regulationssequenzen sind in Promotoren wie cos-, tac-, trp-, tet-, trp-tet-, lpp-, lac-, lpp-lac-, $lacI^{q-,}$ T7-, T5-, T3-, gal-, trc-, ara-, rhaP (rha$P_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare

oder zirkuläre DNA zu verstehen. Diese Vektoren können autonom im Wirtsorganismus repliziert oder chromosomal repliziert werden.

Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, pIN-III$^{113}$-B1, λgt11 oder pBdCl, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, pGHlac$^{+}$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

In einer weiteren Ausgestaltungsform des Vektors kann der das erfindungsgemäß verwendete Nukleinsäurekonstrukt oder die erfindungsgemäß verwendete Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäß verwendeten Nukleinsäure bestehen.

Für eine optimale Expression heterologer Gene in Organismen ist es vorteilhaft die Nukleinsäuresequenzen entsprechend des im Organismus verwendeten spezifischen "Codonnutzung" zu verändern. Der "Codonnutzung" lässt sich anhand von Computerauswertungen anderer, bekannter Gene des betreffenden Organismus leicht ermitteln.

Die Herstellung einer erfindungsgemäß eingesetzenExpressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

### 3. Mikroorganismen

Je nach Zusammenhang kann unter dem Begriff "Mikroorganismus" der Wildtyp-Mikroorganismus oder ein genetisch veränderter, rekombinanter Mikroorganismus oder beides verstanden werden.

Mit Hilfe der erfindungsgemäß verwendeten Vektoren sind rekombinante Mikroorganismen herstellbar, welche beispielsweise mit wenigstens einemsolchen Vektor transformiert sind und zur Produktion der erfindungsgemäß verwendeten Polypeptide eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als rekombinante Wirtsorganismen für die erfindungsgemäß verwendete Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden grampositive oder gram-negative Bakterien, bevorzugt Bakterien der Familien Enterobacteriaceae, Pseudomonadaceae, Rhizobiaceae, Streptomycetaceae oder Nocardiaceae, besonders bevorzugt Bakterien der Gattungen Escherichia, Pseudomonas, Streptomyces, Nocardia, Burkholderia, Salmonella, Agrobacterium, Clostridium oder Rhodococcus verwendet. Ganz besonders bevorzugt ist die Gattung und Art Escherichia coli. Weitere vorteilhafte Bakterien sind darüber hinaus in der Gruppe der alpha-Proteobacterien, beta-Proteobacterien oder gamma-Proteobacterien zu finden.

Der Wirtsorganismus oder die Wirtsorganismen enthalten dabei vorzugsweise mindestens eine der hierin beschriebenen Nukleinsäuresequenzen, Nukleinsäurekonstrukte oder Vektoren, die für ein Enzym mit Phenylethanol Dehydrogenase-Aktivität gemäß obiger Definition kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu Beginn der Fermentation vorgelegt oder semikontinuierlich oder kontinuierlich nachgefüttert werden.

**4. Rekombinante Herstellung von im erfindungsgemäßen Verfahren verwendeten Enzymen**

Hierin beschrieben sind weiterhin Verfahren zur rekombinanten Herstellung erfindungsgemäß verwendeter Enzyme oder funktioneller, biologisch aktiver Fragmente davon, wobei man einen Enzym-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Die so hergestellten Mikroorganismen können kontinuierlich oder diskontinuierlich im batch- Verfahren (Satzkultivierung) oder im fed batch (Zulaufverfahren) oder repeated fed batch Verfahren (repetitives Zulaufverfahren) kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl. Sonnenblumenöl. Erdnußöl und Kokosfett, Fettsäuren wie z.B. Palmitinsäure, Stearinsäure oder Linolsäure, Alkohole wie z. B. Glycerin, Methanol oder Ethanol und organische Säuren wie z.B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15 °C und 45 °C, vorzugsweise bei 25 °C bis 40 °C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z.B. Fettsäurepolyglykolester, eingesetzt werden. Zur Aufrechterhaltung der Stabilität von Plasmiden können dem Medium geeignete selektiv wirkende Stoffe, wie z.B. Antibiotika, hinzugefügt werden. Um aerobe Bedingungen aufrechtzuerhalten, werden Sauerstoff oder Sauerstoff-haltige Gasmischungen, wie z.B. Umgebungsluft, in die Kultur eingetragen. Die Temperatur der Kultur liegt normalerweise bei 20 °C bis 45 °C. Die Kultur wird so lange fortgesetzt, bis sich ein Maximum des gewünschten Produktes gebildet hat. Dieses Ziel wird normalerweise innerhalb von 10 Stunden bis 160 Stunden erreicht.

Die Fermentationsbrühe wird anschließend weiterverarbeitet. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Die Zellen können auch, falls die Polypeptide nicht in das Kulturmedium sezerniert werden, aufgeschlossen und das Produkt nach bekannten Proteinisolierungsverfahren aus dem Lysat gewonnen werden. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, T. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

An analoger Weise können derartige Cyclase-exprimierende Mikroorganismen auch zu fermentativen Herstellung der Verbindungen der Formel II eingesetzt werden, wobei man dem Medium zusätzlich Verbindungen der Formel I als Substrat zusetzt und die Mikroorganismen kultiviert, und das Wertprodukt ggf. aus der Fermentationsbrühe isoliert Darüber hinaus kann die Verbindung der Formel (I) auch von einem Mikroorganismus autonom hergestellt werden, da dieser Mikroorganismus die entsprechende biochemische Ausstattung für die Synthese von (I) aus einfachen Vorstufen besitzt.

## 5. Enzymimmobilisierung

Die erfindungsgemäß verwendeten Enzyme können in den hierin beschriebenen Verfahren frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme

wie auch Zellen können auch direkt mit Glutaraldehyd vernetzt werden (Cross-linking zu CLEAs). Entsprechende und weitere Immobilisierungsverfahren sind beispielsweise in J. Lalonde und A. Margolin "Immobilization of Enzymes" " in K. Drauz und H. Waldmann, Enzyme Catalysis in Organic Synthesis 2002, Vol.III, 991-1032, Wiley-VCH, Weinheim beschrieben. Weitere Informationen zu Biotransformationen und Bioreaktoren zur Durchführung erfindungsgemäßer Verfahren findet man z.B. auch in Rehm et al. (Ed) Biotechology, 2nd Edn, Vol 3, Chapter 17, VCH, Weinheim.

**6. Enzymatische Cyclisierung von Geranyllinalool**

Insbesondere wird das erfindungsgemäße Cyclisierungsverfahren in Gegenwart eines Enzyms durchgeführt, wobei das Enzym von einer Nukleinsäuresequenz gemäß SEQ ID NO:1 oder einem funktionalen Äquivalent davon kodiert wird, wobei die Nukleinsäuresequenz Bestandteil eines Genkonstrukts oder Vektors ist. Solche Genkonstrukte oder Vektoren werden ausführlich in der internationalen Anmeldung PCT/EP2010/057696 auf den Seiten 16 bis 20 beschrieben, worauf hier ausdrücklich Bezug genommen wird.

Die Wirtszelle, die ein Genkonstrukt oder einen Vektor enthält, worin die Nukleinsäuresequenz enthalten ist, die das Enzym mit der gewünschten Aktivität kodiert, bezeichnet man auch als transgenen Organismus. Die Herstellung solcher transgenen Organismen ist prinzipiell bekannt und wird beispielsweise in der internationalen Anmeldung PCT/EP2010/057696 auf Seite 20 diskutiert, worauf hier ausdrücklich Bezug genommen wird.

Als transgene Organismen werden bevorzugt Zellen aus der Gruppe bestehend aus Bakterien, Cyanobakterien, Pilzen und Hefen ausgewählt. Bevorzugt ist die Zelle ausgewählt aus Pilzen der Gattung Pichia oder Bakterien der Gattungen Escherichia, Corynebacterium, Ralstonia, Clostridium, Pseudomonas, Bacillus, Zymomonas, Rhodobacter, Streptomyces, Burkholderia, Lactobacillus oder Lactococcus. Besonders bevorzugt ist die Zelle ausgewählt aus Bakterien der Spezies Escherichia coli, Pseudomonas putida, Burkholderia glumae, Streptomyces lividans, Streptomyces coelicolor oder Zymomonas mobilis.

Bevorzugt ist ein erfindungsgemäßes Verfahren, dadurch gekennzeichnet, dass das Enzym mit der Aktivität einer Cyclase von einem Gen kodiert wird, das aus einem Mikroorganismus isoliert wurde, ausgewählt unter Zymomonas mobilis, isoliert.

Weiterhin bevorzugt ist ein erfindungsgemäßes Verfahren, dadurch gekennzeichnet, dass das Enzym mit der Cyclase-Aktivität von einem Mikroorganismus generiert wurde, der das Enzym überproduziert und der ausgewählt wurde aus der Gruppe der Mikroorganismen bestehend aus den Gattungen Escherichia, Corynebacterium, Ralstonia, Clostridium, Pseudomonas, Bacillus, Zymomonas, Rhodobacter, Streptomyces, Burkholderia, Lactobacillus und Lactococcus.

Besonders zu erwähnen ist ein erfindungsgemäßes Verfahren, dadurch gekennzeichnet dass das Enzym mit der Cyclase-Aktivität von transgenen Mikroorganismen der Spezies Escherichia coli, Pseudomonas putida, Burkholderia glumae, Corynebacterium glutamicum, Saccharomyces cerevisiae, Pichia pastoris, Streptomyces lividans, Streptomyces coelicolor, Bacillus subtilis oder Zymomonas mobilis erzeugt wurde, welche das Enzym mit der Cyclase-Aktivität überproduzieren.

Das erfindungsgemäße Verfahren ist dadurch gekennzeichnet, dass das Enzym in wenigstens einer der folgenden Formen vorliegt:

a) freies, gegebenenfalls gereinigtes oder teilweise gereinigtes Polypeptid;
b) immobilisiertes Polypeptid;
c) aus Zellen isoliertes Polypeptid gemäß a) oder b);
d) ganze Zelle, gegebenenfalls ruhende oder wachsende Zellen, enthaltend mindestens ein solches Polypeptid;
e) Lysat oder Homogenisat der Zellen gemäß d).

Eine weitere Ausführungsform des erfindungsgemäßen Verfahrens ist dadurch gekennzeichnet, dass die Zellen Mikroorganismen sind, bevorzugt transgene Mikroorganismen exprimierend mindestens ein heterologes Nukleinsäuremolekül kodierend für ein Polypeptid mit der Cyclase-Aktivität.

Eine bevorzugte Ausführungsform des erfindungsgemäßen Verfahrens umfasst wenigsten die folgenden Schritte a), b) und d):

a) einen ein Enzym mit Cyclase-Aktivität produzierenden Mikroorganismus aus einer natürlichen Quelle zu isolieren oder rekombinant herzustellen,
b) diesen Mikroorganismus zu vermehren,
c) aus dem Mikroorganismus das Enzym mit Cyclase-Aktivität gegebenenfalls zu isolieren oder eine dieses Enzym enthaltende Proteinfraktion herzustellen, und
d) den Mikroorganismus gemäß Stufe b) oder das Enzym gemäß Stufe c) in ein Medium zu überführen, das Substrat der allgemeinen Formel (I), enthält.

In dem erfindungsgemäßen Verfahren wird Substrat mit dem Cyclase Enzym in einem Medium in Kontakt gebracht und/oder so inkubiert, dass eine Umsetzung des Substrats in Gegenwart des Enzyms erfolgt. Bevorzugt handelt es sich bei dem Medium um ein wässriges Reaktionsmedium.

Der pH-Wert des wässrigen Reaktionsmediums, in dem das erfindungsgemäße Verfahren bevorzugt durchgeführt wird, wird dabei vorteilhaft zwischen pH 4 und 12, bevorzugt zwischen pH 4,5 und 9, besonders bevorzugt zwischen pH 5 und 8 gehalten.

Bei den wässrigen Reaktionsmedien handelt es sich vorzugsweise um gepufferte Lösungen, die in der Regel einen pH-Wert von vorzugsweise von 5 bis 8, aufweisen. Als Puffer kann ein Citrat-, Phosphat-, TRIS- (Tris(hydroxymethyl)-aminomethan) oder MES-Puffer (2-(N-Morpholino)ethansulfonsäure) verwendet werden. Ferner kann das Reaktionsmedium noch weitere Zusätze enthalten, wie z.B. Detergentien (beispielsweise Taurodeoxycholat).

Das Substrat wird vorzugsweise in einer Konzentration von 2 - 200 mM, besonders bevorzugt von 5-25 mM in die enzymatische Umsetzung eingesetzt und kann kontinuierlich oder diskontinuierlich nachgeführt werden.

Die enzymatische Cyclisierung erfolgt in der Regel bei einer Reaktionstemperatur unterhalb der Desaktivierungstemperatur des eingesetzten Enzyms und oberhalb von -10 °C. Bevorzugt wird das erfindungsgemäße Verfahren bei einer Temperatur zwischen 0 °C und 95 °C, besonders bevorzugt bei einer Temperatur zwischen 15 °C und 60 °C, insbesondere zwischen 20 und 45 °C, z.B. bei etwa 25 bis 30°C durchgeführt.

Besonders bevorzugt ist ein erfindungsgemäßes Verfahren, wobei die Umsetzung bei einer Temperatur im Bereich von 20 bis 40°C und/oder einem pH-Wert im Bereich von 4 bis 8 erfolgt.

Neben diesen einphasigen wässrigen Systemen werden in einer weiteren Variante der Erfindung auch zweiphasige Systeme eingesetzt. Dabei werden neben einer wässrigen Phase als zweite Phase, organische, nicht-wassermischbare Reaktionsmedien verwendet. Dadurch akkumulieren die Reaktionsprodukte in der organischen Phase. Nach der Reaktion ist das Produkt in der organische Phase leicht von der wässrigen Phase, die den Biokatalysator enthält, abtrennbar.

Das Reaktionsprodukt kann mit organischen Lösungsmitteln extrahiert werden und gegebenenfalls zur Aufreinigung destilliert werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Heptan, Methyl-tert-butylether, Diisopropylether, Tetrahydrofuran, Ethylacetat verwendet.

Die erfindungsgemäß eingesetzten Cyclasen können im erfindungsgemäßen Verfahren als freies oder immobilisiertes Enzym, wie oben bereits beschrieben, verwendet werden.

Für das erfindungsgemäße Verfahren können ruhende oder wachsende, freie oder immobilisierte Zellen verwendet werden, die für die Cyclase kodierenden Nukleinsäuren, Nukleinsäurekonstrukte oder Vektoren enthalten. Auch aufgeschlossene Zellen, wie Zelllysate oder Zellhomogenate, können verwendet werden. Unter aufgeschlossenen Zellen sind beispielsweise Zellen zu verstehen, die über eine Behandlung beispielsweise mit Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder teilweise gereinigte Enzyme können für das erfindungsgemäße Verfahren verwendet werden.

Werden für das erfindungsgemäße Verfahren freie Organismen oder Enzyme verwendet, so werden diese vor der Extraktion zweckmäßigerweise abgetrennt, beispielsweise über eine Filtration oder Zentrifugation.

Das erfindungsgemäße Verfahren kann batchweise, semi-batchweise oder kontinuierlich betrieben werden.

In einer besonders bevorzugten Ausführungsform des erfindungsgemäßen Verfahrens ist das Enzym mit Cyclase-Aktivität ausgewählt unter Enzymen, welche eine Aminosäuresequenz gemäß SEQ ID NO: 2 umfassen oder eine davon abgeleitete Sequenz, bei der bis zu 5, 4, 3, 2, 1 % der Aminosäurereste durch eine Deletion, eine Substitution eine Insertion oder eine Kombination aus Deletion, Substitution und Insertion verändert worden sind. Dabei können diese gegenüber SEQ ID NO: 2 veränderten Polypeptidsequenzen noch mindestens 50 %, bevorzugt 65 %, besonders bevorzugt 80 % , insbesondere mehr als 90 % der enzymatischen Aktivität von SEQ ID NO:2 besitzen. In diesem Zusammenhang soll unter enzymatischer Aktivität von SEQ ID NO:2 die Fähigkeit verstanden werden, eine Verbindung der allgemeinen Formel (I) biokatalytisch zu dem entsprechenden Verbindung der Formel (II) zu cyclisieren.

## Experimenteller Teil

Sofern keine speziellen Angaben in den folgenden Beispielen gemacht werden, gelten die folgenden allgemeinen Angaben.

### A. Allgemeine Angaben

Sämtliche eingesetzte Materialien und Mikroorganismen sind im Handel erhältliche Produkte.

Soweit nicht anderes angegeben wird, erfolgt die Klonierung und Expression von rekombinanten Proteinen nach Standardmethoden, wie z.B. in Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

#### a) Bakterienstämme, Plasmide und Wachstumsbedingungen

Aus einer entsprechenden 2mL Vorkultur angeimpft, wurde *E. coli* LU15568 in 20mL Lytic Broth-Amp/Spec/Cm (100µg/l Ampicillin; 100µg/l Spectinomycin; 20µg/l Chloramphenicol), in Gegenwart von 0,1 mM IPTG, 0,5g/L Rhamnose in 100mL Erlenmeyerkolben (mit Schikanen) 16 h bei 37°C angezogen, bei 5000**g* für 10min zentrifugiert.

#### b) Cyclisierungs-Assay mit Geranyllinalool (Standardbedingungen)

Rekombinante *E. coli* Zellen wurden in 20 mM Tris-HCl pH 8.0 (3 ml pro g feuchter Zellen) suspendiert. Das Zyklisierungsgemisch enthielt 250 µl Zellsuspension, 50 µl 1 M Citratpuffer (pH 4.5), 20 mM (Endkonzentration) Substrat und Wasser ad 500 µl. Bei Zyklisierung von Squalen wurde 1 % (v/v) Triton-X100 zugesetzt. Für die Zyklisierungsreaktion wurden *E. coli* Zellen (6 g feuchte Zellen) in Solubilisierungspuffer (50 mM Phosphat, 10 mM $MgCl_2$ (pH 6,5; Gesamtvolumen: 25 ml) suspendiert. Die Zellen wurden bei 1500 bar unter Verwendung eines Manton-Gaulin Homogenisators aufgeschlossen. Unlösliche Zelldebris wurde abzentrifugiert (15 min bei 4 °C and 7150*g).

Für die Umsetzung von Geranyllinalool wurden 1,2 mL $KP_i$-Puffer (50 mM Kaliumphosphat, pH 6,5; 10 mM $MgCl_2$) mit 1 mL Enzymrohextrakt (Proteingehalt 39,3 mg/mL in 50 mM $KP_i$-Puffer) und 22 µL Geranyllinalool (Fa. Sigma-Aldrich-Fluka; Best.Nr. 48809) gemischt und in einem 10 mL Schraubdeckelglas mit Magnetrührstab drei bzw. zwanzig Stunden bei 37 °C und 300 U/min inkubiert.

Am Ende der Inkubationszeit wurden die Proben mit 5 mL n-Heptan / 1-Propanol (3:2) extrahiert und mittels GC analysiert. Kontrollen wurden mit *E. coli* Zellen durchgeführt, die einen leeren Vektor trugen sowie mit Hitze-inaktivierten SHC-exprimierenden Zellen.

#### c) Gaschromatographie

| | |
|---|---|
| Apparatur: | Agilent 6890series |
| Säule: | OPTIMA-1 TG ID: 0,32 mm, Länge: 10 m (Macherey-Nagel, Düren, Deutschland) |
| Flussrate: | 1.0/min at 5.1 psi (und 80 °C) |
| Split: | 1:50, Splitflow: 50 mL/min, |
| Träger: | Stickstoff |
| Injector: | split/splitless liner (Restec GmbH, Bad Homburg, Deutschland; Siltec-deaktiviert, 4*6.3*78.5 mm, Glaswolle) injeKtortemperatur 280 °C |
| Injektionsvolumen: | 1 µL |
| Detektor: | FID mit 300 mL/min Luft, 30 mL/min Wasserstoff und 30 mL/min Stickstoff |
| Detektortemperatur: | 320 °C |

Temperaturprogramm:

| | |
|---|---|
| Start: | 100 °C |
| Haltezeit 1: | 0 min |
| T-Anstieg 1: | 5°C/min |
| T-End 1: | 200 °C |

(fortgesetzt)

| | |
|---|---|
| Haltezeit 2: | 5 min |
| T-Anstieg 2: | 30 °C/min |
| T-End 2: | 320 °C |
| Haltezeit 3: | 20 min |
| Gesamtzeit: | 49.0 min |
| Datenanalyse: | Empower-3-Software Service Release 1 (Waters GmbH, Eschborn, Deutschland) |

Retentionszeit Geranyllinalool: 14,4 min

**B. Beispiele**

**Beispiel 1: Cyclsierung von Geranyllinalool durch Zmo-SHC (SEQ ID NO:2)**

**a) Durchführung:**

Rekombinante Zmo-SHC wurde wie beschrieben durch Anzucht und Aufschluss von LU 15568 hergestellt. (vgl. PCT/EP2010/057696 (WO2010139719 A2) Um diese Enzympräparation zu überprüfen wurde mit Homofarnesol als Referenzsubstrat die Aktivität erneut bestimmt. Die erhaltene Aktivität (63 % Umsatz in 3 Stunden mit 39 mg Gesamtprotein) liegt im Rahmen der bisher unter diesen Bedingungen erzielten Ergebnisse.

Die Umsetzung von Geranyllinalool erfolgte wie oben beschrieben unter Standardbedingungen.

Am Ende der Inkubationszeit wurden die Proben mit 5 mL n-Heptan / 1-Propanol (3:2) extrahiert und mittels GC analysiert.

Reaktionsgleichung:

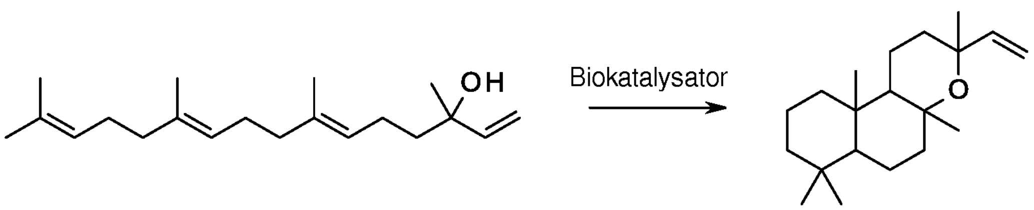


(**1**)
***E,E*-Geranyllinalool**
(6*E*,10*E*)-3,7,11,15-tetramethylhexadeca-1,6,10,14-tetraen-3-ol

(**2**)
3,4a,7,7,10a-Pentamethyl-3-vinyl-2,5,6,6a,8,9,10,10b-octahydro-1H-benzo[f]chromen

**b) Ergebnis**

Nach 20 Stunden wurden zwei Enzym-abhängige Peaks (Retentionszeit 13,3 bzw. 13,7 min) gefunden. Der Umsatz, bezogen auf das eingesetzte Geranyllinalool, beträgt rund 11 %. Mittels GC-MS Analyse und Interpretation der Massenspektren wurde den beiden Peaks zwei Strukturisomeren des Benzochromenderivats 2 zugeordnet. Die Unterschiede zwischen den beiden Peaks bei 13,3 bzw. 13,7 min konnte mittels MS nicht aufgeklärt werden. Es ist beispielsweise möglich, dass es sich um Stereoisomere der Verbindung 2 handelt.

In einer Negativkontrolle, die ohne Enzymzugabe inkubiert wurde, konnten diese Peaks nicht detektiert werden.

Dieses Ergebnis zeigt, dass die Zmo-SHC auch in der Lage ist, Geranyllinalool (1) zu zyklisieren. Allerdings scheinen bei der Reaktion mindestens zwei Isomere aufzutreten.

**c) Zusammenfassung:**

Mit der rekombinanten Squalen-Hopen-Cyclase aus *Zymomonas mobilis* konnte erstmals die Zyklisierung von Geranyllinalool zu einem trizyklischen Vinylbenzochromenderivat gezeigt werden.

**Sequenzen:**

SEQ ID NO: 1: Nukleinsäuresequenz der Zmo-SHC
SEQ ID NO: 2: Aminosäuresequenzen der Zmo-SHC

## Patentansprüche

1. Verfahren zur biokatalytischen Cyclisierung einer Verbindung der Formel (I)

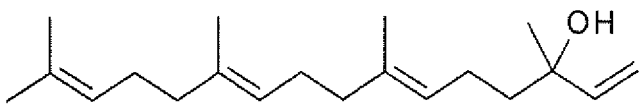

(I)

in Gegenwart einer Cyclase, die eine Aminosäuresequenz gemäß SEQ ID NO:2 oder mit wenigstens 95% Sequenzidentität zu SEQ ID NO:2 aufweist;
wobei man die Verbindung der Formel (I) zu der Verbindung der Formel (II)

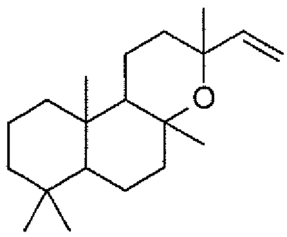

(II)

umsetzt.

2. Verfahren nach Anspruch 1, wobei die Cyclase roh, gereinigt, gelöst, dispergiert oder immobilisiert vorliegt, oder in Gegenwart von Cyclase aufweisenden Zellen eines Mikroorganismus.

3. Verfahren nach einem der Ansprüche 1 und 2 in Gegenwart eines rekombinanten Mikroorganismus, der eine für die Cyclase kodierende Nukleotidsequenz trägt.

4. Verfahren nach Anspruch 3, wobei die Nukleotidsequenz Teil einer Expressionskassette ist und darin unter der Kontrolle wenigstens einer regulativen Sequenz steht.

5. Verfahren nach Anspruch 4, wobei die Expressionskassette Teil eines Expressionsvektors ist.

6. Verfahren nach Anspruch 5, wobei der Expressionsvektor unter Plasmiden ausgewählt ist.

7. Verfahren nach einem der Ansprüche 2 bis 6, wobei der Mikroorganismus unter Bakterien, Pilzen und Hefen ausgewählt ist.

8. Verfahren nach Anspruch 7, wobei der Mikroorganismus *E. coli* ist.

## Claims

1. A process for the biocatalytic cyclization of a compound of the formula (I)

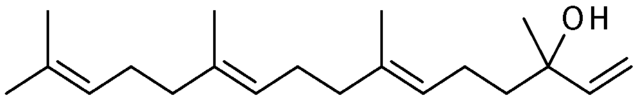

(I)

in the presence of a cyclase which has an amino acid sequence as shown in SEQ ID No. 2 or which has at least 95% sequence identity to SEQ ID No. 2; wherein the compound of the formula (I) is reacted to give the compound of the formula (II)

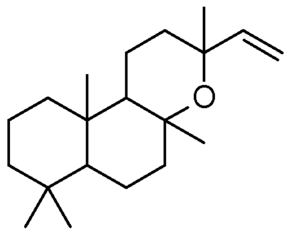

(II).

2. The process according to claim 1, wherein the cyclase is present in crude, purified, dissolved, dispersed or immobilized form, or in the presence of cyclase- displaying cells of a microorganism.

3. The process according to either of claims 1 and 2 in the presence of a recombinant microorganism which includes a nucleotide sequence which codes for the cyclase.

4. The process according to claim 3, wherein the nucleotide sequence is part of an expression cassette and, therein, is under the control of at least one regulatory sequence.

5. The process according to claim 4, wherein the expression cassette is part of an expression vector.

6. The process according to claim 5, wherein the expression vector is selected from among plasmids.

7. The process according to any of claims 2 to 6, wherein the microorganism is selected from among bacteria, fungi and yeasts.

8. The process according to claim 7, wherein the microorganism is *E. coli.*

## Revendications

1. Procédé de cyclisation biocatalytique d'un composé de formule (I)

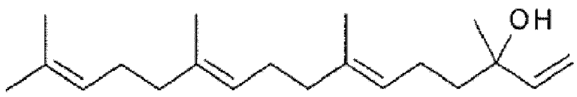

(I)

en présence d'une cyclase qui présente une séquence d'acides aminés selon SEQ ID NO : 2 ou qui présente une identité de séquence d'au moins 95 % avec SEQ ID NO : 2 ;
le composé de formule (I) étant transformé en le composé de formule (II)

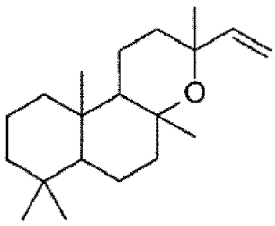

(II)

.

**2.** Procédé selon la revendication 1, dans lequel la cyclase se présente sous forme brute, purifiée, dissoute, dispersée ou immobilisée, ou en présence de cellules d'un microorganisme comprenant de la cyclase.

**3.** Procédé selon l'une quelconque des revendications 1 et 2, en présence d'un microorganisme recombinant qui porte une séquence nucléotidique codant pour la cyclase.

**4.** Procédé selon la revendication 3, dans lequel la séquence nucléotidique fait partie d'une cassette d'expression et s'y trouve sous le contrôle d'au moins une séquence de régulation.

**5.** Procédé selon la revendication 4, dans lequel la cassette d'expression fait partie d'un vecteur d'expression.

**6.** Procédé selon la revendication 5, dans lequel le vecteur d'expression est choisi parmi les plasmides.

**7.** Procédé selon l'une quelconque des revendications 2 à 6, dans lequel le microorganisme est choisi parmi les bactéries, les champignons et les levures.

**8.** Procédé selon la revendication 7, dans lequel le microorganisme est *E. coli.*

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2011070304 W
- WO 2012066059 A2
- EP 2010057696 W
- WO 2010139719 A2
- EP 1149849 A
- EP 1069183 A
- DE OS100193773 A
- LU 15568


### In der Beschreibung aufgeführte Nicht-Patentliteratur


- Abspaltung eines Protons, zu einem zyklischen Monoterpen rekombiniert. *Curr. Opin. Chem. Biol.,* 2009, vol. 13, 180-188
- **SIEDENBURG, G. ; JENDROSSEK, D.** *Applied and Environmental Microbiology,* 2011, vol. 77 (12), 3905
- *Nat Biotechnol,* 2005, vol. 23, 63-68
- *Enzymcode gemäß Eur. J. Biochem.,* 1999, vol. 264, 610-650
- **SOBOLEV V ; SOROKINE A ; PRILUSKY J ; ABOLA EE ; EDELMAN M.** Automated analysis of interatomic contacts in proteins. *Bioinformatics,* 1999, vol. 15 (4), 327-332, http://ligin.weizmann.ac.il/cgibin/lpccsu/LpcCsu.cgi
- **NARANG, S.A.** *Tetrahedron,* 1983, vol. 39, 3
- **ITAKURA et al.** *Annu. Rev. Biochem.,* 1984, vol. 53, 323
- **ITAKURA et al.** *Science,* 1984, vol. 198, 1056
- **IKE et al.** *Nucleic Acids Res.,* 1983, vol. 11, 477
- **ARKIN ; YOURVAN.** *PNAS,* 1992, vol. 89, 7811-7815
- **DELGRAVE et al.** *Protein Engineering,* 1993, vol. 6 (3), 327-331
- **PEARSON ; LIPMAN.** *Proc. Natl. Acad, Sci. (USA),* 1988, vol. 85 (8), 2444-2448
- **VOET ; VOET.** Phosphoamiditmethode. Wiley Press, 896-897
- **SAMBROOK et al.** Molecular Cloning: A laboratory manual. Cold Spring Harbor Laboratory Press, 1989
- **SAMBROOK, J. ; FRITSCH, E.F. ; MANIATIS, T.** Molecular Cloning: A Laboratory Manual. Cold Spring Harbor Laboratory Press, 1989
- **SAMBROOK et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **SAMBROOK, J. ; FRITSCH, E.F. ; MANIATIS, T.** Molecular Cloning (A Laboratory Manual). Cold Spring Harbor Laboratory Press, 1989, 9.31-9.57
- Current Protocols in Molecular Biology. John Wiley & Sons, 1989, 6.3.1-6.3.6
- **HIGGINS DG ; SHARP PM.** Fast and sensitive multiple sequence alignments on a microcomputer. *Comput Appl. Biosci.,* April 1989, vol. 5 (2), 151-1
- **CHENNA ; RAMU ; SUGAWARA ; HIDEAKI ; KOIKE ; TADASHI ; LOPEZ ; RODRIGO ; GIBSON ; TOBY J.** Multiple sequence alignment with the Clustal series of programs. *Nucleic Acids Res,* 2003, vol. 31 (13), 3497-500
- **SAMBROOK ; RUSSELL.** Molecular Cloning. Cold Spring Harbor Laboratory Press, 2001
- In vitro mutagenesis protocols. Humana Press, 1996
- **KEGLER-EBO DM ; DOCKTOR CM ; DIMAIO D.** *Nucleic Acids Res,* 1994, vol. 22, 1593
- **BARETTINO D ; FEIGENBUTZ M ; VALCÁREL R ; STUNNENBERG HG.** *Nucleic Acids Res,* 1994, vol. 22, 541
- **BARIK S.** *Mol Biotechnol,* 1995, vol. 3, 1
- **ECKERT KA ; KUNKEL TA.** *Nucleic Acids Res,* 1990, vol. 18, 3739
- **SCHENK et al.** Sequence Saturation Method), bei der bevorzugte Austausche durch die Polymerase verhindert werden. *Biospektrum,* 2006, vol. 3, 277-279
- An efficient random mutagenesis technique using an E.coli mutator strain. **GREENER A ; CALLAHAN M ; JERPSETH B.** In vitro mutagenesis protocols. Humana Press, 1996
- **STEMMER WPC.** *Nature,* 1994, vol. 370, 389
- **STEMMER WPC.** *Proc Natl Acad Sci USA,* 1994, vol. 91, 10747

- **REETZ MT ; JAEGER K-E.** Topics Curr Chem. 1999, vol. 200, 31
- Methods for optimizing industrial enzymes by directed evolution. **ZHAO H ; MOORE JC ; VOLKOV AA ; ARNOLD FH.** Manual of industrial microbiology and biotechnology. American Society for Microbiology, 1999
- **GOEDDEL.** Gene Expression Technology: Methods in Enzymology. Academic Press, 1990, vol. 185
- Buch Cloning Vectors. Elsevier, 1985
- **T. MANIATIS ; E.F. FRITSCH ; J. SAMBROOK.** Molecular Cloning: A Laboratory Manual. Cold Spring Harbor Laboratory, 1989
- **T.J. SILHAVY ; M.L. BERMAN ; L.W. ENQUIST.** Experiments with Gene Fusions. Cold Spring Harbor Laboratory, 1984
- **AUSUBEL, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **SAMBROOK et al.** Molecular Cloning: A Laboratory Manual. Cold Spring Harbor Laboratory Press, 1989
- Bioprozeßtechnik 1. Einführung in die Bioverfahrenstechnik. Gustav Fischer Verlag, 1991
- Bioreaktoren und periphere Einrichtungen. Vieweg Verlag, 1994
- Manual of Methods für General Bacteriology. American Society für Bacteriology, 1981
- Applied Microbiol. Physiology, A Practical Approach. IRL Press, 1997, 53-73
- **HARLOW, E. ; LANE, D.** Antibodies: A Laboratory Manual. Cold Spring Harbor, 1988
- Immobilization of Enzymes. **J. LALONDE ; A. MARGOLIN.** Enzyme Catalysis in Organic Synthesis. Wiley-VCH, 2002, vol. III, 991-1032
- Biotechology. VCH, vol. 3
- **SAMBROOK, J. ; FRITSCH, E.F. ; MANIATIS, T.** Molecular cloning: A Laboratory Manual. Cold Spring Harbor Laboratory Press, 1989

SEQUENCE LISTING

<110> BASF SE

<120> Enzymatische Cyclisierung von Geranyllinalool

<130> PF 75027, M/54304

<160> 2

<170> PatentIn version 3.5

<210> 1
<211> 2178
<212> DNA
<213> Zymomonas mobilis

<220>
<221> CDS
<222> (1)..(2178)

<400> 1

```
atg ggt att gac aga atg aat agc tta agt cgc ttg tta atg aag aag     48
Met Gly Ile Asp Arg Met Asn Ser Leu Ser Arg Leu Leu Met Lys Lys
1               5                   10                  15

att ttc ggg gct gaa aaa acc tcg tat aaa ccg gct tcc gat acc ata     96
Ile Phe Gly Ala Glu Lys Thr Ser Tyr Lys Pro Ala Ser Asp Thr Ile
            20                  25                  30

atc gga acg gat acc ctg aaa aga ccg aac cgg cgg cct gaa ccg acg    144
Ile Gly Thr Asp Thr Leu Lys Arg Pro Asn Arg Arg Pro Glu Pro Thr
        35                  40                  45

gca aaa gtc gac aaa acg ata ttc aag act atg ggg aat agt ctg aat    192
Ala Lys Val Asp Lys Thr Ile Phe Lys Thr Met Gly Asn Ser Leu Asn
    50                  55                  60

aat acc ctt gtt tca gcc tgt gac tgg ttg atc gga caa caa aag ccc    240
Asn Thr Leu Val Ser Ala Cys Asp Trp Leu Ile Gly Gln Gln Lys Pro
65                  70                  75                  80

gat ggt cat tgg gtc ggt gcc gtg gaa tcc aat gct tcg atg gaa gca    288
Asp Gly His Trp Val Gly Ala Val Glu Ser Asn Ala Ser Met Glu Ala
                85                  90                  95

gaa tgg tgt ctg gcc ttg tgg ttt ttg ggt ctg gaa gat cat ccg ctt    336
Glu Trp Cys Leu Ala Leu Trp Phe Leu Gly Leu Glu Asp His Pro Leu
            100                 105                 110

cgt cca aga ttg ggc aat gct ctt ttg gaa atg cag cgg gaa gat ggc    384
Arg Pro Arg Leu Gly Asn Ala Leu Leu Glu Met Gln Arg Glu Asp Gly
        115                 120                 125

tct tgg gga gtc tat ttc ggc gct gga aat ggc gat atc aat gcc acg    432
Ser Trp Gly Val Tyr Phe Gly Ala Gly Asn Gly Asp Ile Asn Ala Thr
    130                 135                 140

gtt gaa gcc tat gcg gcc ttg cgg tct ttg ggg tat tct gcc gat aat    480
Val Glu Ala Tyr Ala Ala Leu Arg Ser Leu Gly Tyr Ser Ala Asp Asn
145                 150                 155                 160
```

```
cct gtt ttg aaa aaa gcg gca gca tgg att gct gaa aaa ggc gga tta        528
Pro Val Leu Lys Lys Ala Ala Ala Trp Ile Ala Glu Lys Gly Gly Leu
                165                 170                 175

aaa aat atc cgt gtc ttt acc cgt tat tgg ctg gcg ttg atc ggg gaa        576
Lys Asn Ile Arg Val Phe Thr Arg Tyr Trp Leu Ala Leu Ile Gly Glu
            180                 185                 190

tgg cct tgg gaa aag acc cct aac ctt ccc cct gaa att atc tgg ttc        624
Trp Pro Trp Glu Lys Thr Pro Asn Leu Pro Pro Glu Ile Ile Trp Phe
        195                 200                 205

cct gat aat ttt gtc ttt tcg att tat aat ttt gcc caa tgg gcg cgg        672
Pro Asp Asn Phe Val Phe Ser Ile Tyr Asn Phe Ala Gln Trp Ala Arg
    210                 215                 220

gca acc atg gtg ccg att gct att ctg tcc gcg aga cga cca agc cgc        720
Ala Thr Met Val Pro Ile Ala Ile Leu Ser Ala Arg Arg Pro Ser Arg
225                 230                 235                 240

ccg ctg cgc cct caa gac cga ttg gat gaa ctg ttt cca gaa ggc cgc        768
Pro Leu Arg Pro Gln Asp Arg Leu Asp Glu Leu Phe Pro Glu Gly Arg
                245                 250                 255

gct cgc ttt gat tat gaa ttg ccg aaa aaa gaa ggc atc gat ctt tgg        816
Ala Arg Phe Asp Tyr Glu Leu Pro Lys Lys Glu Gly Ile Asp Leu Trp
            260                 265                 270

tcg caa ttt ttc cga acc act gac cgt gga tta cat tgg gtt cag tcc        864
Ser Gln Phe Phe Arg Thr Thr Asp Arg Gly Leu His Trp Val Gln Ser
        275                 280                 285

aat ctg tta aag cgc aat agc ttg cgt gaa gcc gct atc cgt cat gtt        912
Asn Leu Leu Lys Arg Asn Ser Leu Arg Glu Ala Ala Ile Arg His Val
    290                 295                 300

ttg gaa tgg att atc cgg cat cag gat gcc gat ggc ggt tgg ggt gga        960
Leu Glu Trp Ile Ile Arg His Gln Asp Ala Asp Gly Gly Trp Gly Gly
305                 310                 315                 320

att cag cca cct tgg gtc tat ggt ttg atg gcg tta cat ggt gaa ggc       1008
Ile Gln Pro Pro Trp Val Tyr Gly Leu Met Ala Leu His Gly Glu Gly
                325                 330                 335

tat cag ctt tat cat ccg gtg atg gcc aag gct ttg tcg gct ttg gat       1056
Tyr Gln Leu Tyr His Pro Val Met Ala Lys Ala Leu Ser Ala Leu Asp
            340                 345                 350

gat ccc ggt tgg cga cat gac aga ggc gag tct tct tgg ata cag gcc       1104
Asp Pro Gly Trp Arg His Asp Arg Gly Glu Ser Ser Trp Ile Gln Ala
        355                 360                 365

acc aat agt ccg gta tgg gat aca atg ttg gcc ttg atg gcg tta aaa       1152
Thr Asn Ser Pro Val Trp Asp Thr Met Leu Ala Leu Met Ala Leu Lys
    370                 375                 380

gac gcc aag gcc gag gat cgt ttt acg ccg gaa atg gat aag gcc gcc       1200
Asp Ala Lys Ala Glu Asp Arg Phe Thr Pro Glu Met Asp Lys Ala Ala
385                 390                 395                 400

gat tgg ctt ttg gct cga cag gtc aaa gtc aaa ggc gat tgg tca atc       1248
Asp Trp Leu Leu Ala Arg Gln Val Lys Val Lys Gly Asp Trp Ser Ile
                405                 410                 415
```

```
aaa ctg ccc gat gtt gaa ccc ggt gga tgg gca ttt gaa tat gcc aat        1296
Lys Leu Pro Asp Val Glu Pro Gly Gly Trp Ala Phe Glu Tyr Ala Asn
            420                 425                 430

gat cgc tat ccc gat acc gat gat acc gcc gtc gct ttg atc gcc ctt        1344
Asp Arg Tyr Pro Asp Thr Asp Asp Thr Ala Val Ala Leu Ile Ala Leu
        435                 440                 445

tcc tct tat cgt gat aag gag gag tgg caa aag aaa ggc gtt gag gac        1392
Ser Ser Tyr Arg Asp Lys Glu Glu Trp Gln Lys Lys Gly Val Glu Asp
    450                 455                 460

gcc att acc cgt ggg gtt aat tgg ttg atc gcc atg caa agc gaa tgt        1440
Ala Ile Thr Arg Gly Val Asn Trp Leu Ile Ala Met Gln Ser Glu Cys
465                 470                 475                 480

ggc ggt tgg gga gcc ttt gat aag gat aat aac aga agt atc ctt tcc        1488
Gly Gly Trp Gly Ala Phe Asp Lys Asp Asn Asn Arg Ser Ile Leu Ser
                485                 490                 495

aaa att cct ttt tgt gat ttc gga gaa tct att gat ccg cct tca gtc        1536
Lys Ile Pro Phe Cys Asp Phe Gly Glu Ser Ile Asp Pro Pro Ser Val
            500                 505                 510

gat gta acg gcg cat gtt tta gag gcc ttt ggc acc ttg gga ctg tcc        1584
Asp Val Thr Ala His Val Leu Glu Ala Phe Gly Thr Leu Gly Leu Ser
        515                 520                 525

cgc gat atg ccg gtc atc caa aaa gcg atc gac tat gtc cgt tcc gaa        1632
Arg Asp Met Pro Val Ile Gln Lys Ala Ile Asp Tyr Val Arg Ser Glu
    530                 535                 540

cag gaa gcc gaa ggc gcg tgg ttt ggt cgt tgg ggc gtt aat tat atc        1680
Gln Glu Ala Glu Gly Ala Trp Phe Gly Arg Trp Gly Val Asn Tyr Ile
545                 550                 555                 560

tat ggc acc ggt gcg gtt ctg cct gct ttg gcg gcg atc ggt gaa gat        1728
Tyr Gly Thr Gly Ala Val Leu Pro Ala Leu Ala Ala Ile Gly Glu Asp
                565                 570                 575

atg acc cag cct tac atc acc aag gct tgc gat tgg ctg gtc gca cat        1776
Met Thr Gln Pro Tyr Ile Thr Lys Ala Cys Asp Trp Leu Val Ala His
            580                 585                 590

cag cag gaa gac ggc ggt tgg ggc gaa agc tgc tct tcc tat atg gag        1824
Gln Gln Glu Asp Gly Gly Trp Gly Glu Ser Cys Ser Ser Tyr Met Glu
        595                 600                 605

att gat tcc att ggg aag ggc cca acc acg ccg tcc cag act gct tgg        1872
Ile Asp Ser Ile Gly Lys Gly Pro Thr Thr Pro Ser Gln Thr Ala Trp
    610                 615                 620

gct ttg atg ggg ttg atc gcg gcc aat cgt ccc gaa gat tat gaa gcc        1920
Ala Leu Met Gly Leu Ile Ala Ala Asn Arg Pro Glu Asp Tyr Glu Ala
625                 630                 635                 640

att gcc aag gga tgc cat tat ctg att gat cgc caa gag cag gat ggt        1968
Ile Ala Lys Gly Cys His Tyr Leu Ile Asp Arg Gln Glu Gln Asp Gly
                645                 650                 655

agc tgg aaa gaa gaa gaa ttc acc ggc acc gga ttc ccc ggt tat ggc        2016
Ser Trp Lys Glu Glu Glu Phe Thr Gly Thr Gly Phe Pro Gly Tyr Gly
```

```
            660                 665                 670

gtg ggt cag acg atc aag ttg gat gat ccg gct tta tcg aaa cga ttg      2064
Val Gly Gln Thr Ile Lys Leu Asp Asp Pro Ala Leu Ser Lys Arg Leu
        675                 680                 685

ctt caa ggc gct gaa ctg tca cgg gcg ttt atg ctg cgt tat gat ttt      2112
Leu Gln Gly Ala Glu Leu Ser Arg Ala Phe Met Leu Arg Tyr Asp Phe
    690                 695                 700

tat cgg caa ttc ttc ccg att atg gcg tta agt cgg gca gag aga ctg      2160
Tyr Arg Gln Phe Phe Pro Ile Met Ala Leu Ser Arg Ala Glu Arg Leu
705                 710                 715                 720

att gat ttg aat aat tga                                              2178
Ile Asp Leu Asn Asn
                725
```

<210> 2
<211> 725
<212> PRT
<213> Zymomonas mobilis

<400> 2

```
Met Gly Ile Asp Arg Met Asn Ser Leu Ser Arg Leu Leu Met Lys Lys
1               5                   10                  15

Ile Phe Gly Ala Glu Lys Thr Ser Tyr Lys Pro Ala Ser Asp Thr Ile
            20                  25                  30

Ile Gly Thr Asp Thr Leu Lys Arg Pro Asn Arg Arg Pro Glu Pro Thr
        35                  40                  45

Ala Lys Val Asp Lys Thr Ile Phe Lys Thr Met Gly Asn Ser Leu Asn
    50                  55                  60

Asn Thr Leu Val Ser Ala Cys Asp Trp Leu Ile Gly Gln Gln Lys Pro
65                  70                  75                  80

Asp Gly His Trp Val Gly Ala Val Glu Ser Asn Ala Ser Met Glu Ala
                85                  90                  95

Glu Trp Cys Leu Ala Leu Trp Phe Leu Gly Leu Glu Asp His Pro Leu
            100                 105                 110

Arg Pro Arg Leu Gly Asn Ala Leu Leu Glu Met Gln Arg Glu Asp Gly
        115                 120                 125

Ser Trp Gly Val Tyr Phe Gly Ala Gly Asn Gly Asp Ile Asn Ala Thr
    130                 135                 140

Val Glu Ala Tyr Ala Ala Leu Arg Ser Leu Gly Tyr Ser Ala Asp Asn
```

```
145                 150                 155                 160

Pro Val Leu Lys Lys Ala Ala Ala Trp Ile Ala Glu Lys Gly Gly Leu
                165                 170                 175

Lys Asn Ile Arg Val Phe Thr Arg Tyr Trp Leu Ala Leu Ile Gly Glu
            180                 185                 190

Trp Pro Trp Glu Lys Thr Pro Asn Leu Pro Pro Glu Ile Ile Trp Phe
        195                 200                 205

Pro Asp Asn Phe Val Phe Ser Ile Tyr Asn Phe Ala Gln Trp Ala Arg
    210                 215                 220

Ala Thr Met Val Pro Ile Ala Ile Leu Ser Ala Arg Arg Pro Ser Arg
225                 230                 235                 240

Pro Leu Arg Pro Gln Asp Arg Leu Asp Glu Leu Phe Pro Glu Gly Arg
                245                 250                 255

Ala Arg Phe Asp Tyr Glu Leu Pro Lys Lys Glu Gly Ile Asp Leu Trp
            260                 265                 270

Ser Gln Phe Phe Arg Thr Thr Asp Arg Gly Leu His Trp Val Gln Ser
        275                 280                 285

Asn Leu Leu Lys Arg Asn Ser Leu Arg Glu Ala Ala Ile Arg His Val
    290                 295                 300

Leu Glu Trp Ile Ile Arg His Gln Asp Ala Asp Gly Gly Trp Gly Gly
305                 310                 315                 320

Ile Gln Pro Pro Trp Val Tyr Gly Leu Met Ala Leu His Gly Glu Gly
                325                 330                 335

Tyr Gln Leu Tyr His Pro Val Met Ala Lys Ala Leu Ser Ala Leu Asp
            340                 345                 350

Asp Pro Gly Trp Arg His Asp Arg Gly Glu Ser Ser Trp Ile Gln Ala
        355                 360                 365

Thr Asn Ser Pro Val Trp Asp Thr Met Leu Ala Leu Met Ala Leu Lys
    370                 375                 380

Asp Ala Lys Ala Glu Asp Arg Phe Thr Pro Glu Met Asp Lys Ala Ala
385                 390                 395                 400
```

Asp Trp Leu Leu Ala Arg Gln Val Lys Val Lys Gly Asp Trp Ser Ile
405 410 415

Lys Leu Pro Asp Val Glu Pro Gly Gly Trp Ala Phe Glu Tyr Ala Asn
420 425 430

Asp Arg Tyr Pro Asp Thr Asp Asp Thr Ala Val Ala Leu Ile Ala Leu
435 440 445

Ser Ser Tyr Arg Asp Lys Glu Glu Trp Gln Lys Lys Gly Val Glu Asp
450 455 460

Ala Ile Thr Arg Gly Val Asn Trp Leu Ile Ala Met Gln Ser Glu Cys
465 470 475 480

Gly Gly Trp Gly Ala Phe Asp Lys Asp Asn Asn Arg Ser Ile Leu Ser
485 490 495

Lys Ile Pro Phe Cys Asp Phe Gly Glu Ser Ile Asp Pro Pro Ser Val
500 505 510

Asp Val Thr Ala His Val Leu Glu Ala Phe Gly Thr Leu Gly Leu Ser
515 520 525

Arg Asp Met Pro Val Ile Gln Lys Ala Ile Asp Tyr Val Arg Ser Glu
530 535 540

Gln Glu Ala Glu Gly Ala Trp Phe Gly Arg Trp Gly Val Asn Tyr Ile
545 550 555 560

Tyr Gly Thr Gly Ala Val Leu Pro Ala Leu Ala Ala Ile Gly Glu Asp
565 570 575

Met Thr Gln Pro Tyr Ile Thr Lys Ala Cys Asp Trp Leu Val Ala His
580 585 590

Gln Gln Glu Asp Gly Gly Trp Gly Glu Ser Cys Ser Ser Tyr Met Glu
595 600 605

Ile Asp Ser Ile Gly Lys Gly Pro Thr Thr Pro Ser Gln Thr Ala Trp
610 615 620

Ala Leu Met Gly Leu Ile Ala Ala Asn Arg Pro Glu Asp Tyr Glu Ala
625 630 635 640

Ile Ala Lys Gly Cys His Tyr Leu Ile Asp Arg Gln Glu Gln Asp Gly
645 650 655

```
Ser Trp Lys Glu Glu Glu Phe Thr Gly Thr Gly Phe Pro Gly Tyr Gly
            660                 665                 670

Val Gly Gln Thr Ile Lys Leu Asp Asp Pro Ala Leu Ser Lys Arg Leu
        675                 680                 685

Leu Gln Gly Ala Glu Leu Ser Arg Ala Phe Met Leu Arg Tyr Asp Phe
    690                 695                 700

Tyr Arg Gln Phe Phe Pro Ile Met Ala Leu Ser Arg Ala Glu Arg Leu
705                 710                 715                 720

Ile Asp Leu Asn Asn
                725
```